# EUROPEAN PATENT APPLICATION

(11) **EP 2 314 578 A1**
(43) Date of publication of application: **27.04.2011**
(21) Application number: 09382203.9
(22) Date of filing: 13.10.2009
(51) Int. Cl.: C07D 231/14, C07D 401/06, A61P 3/04, A61K 31/415

(54) **Cinchonidine salt of (4S,5S)-5-(4-chlorophenyl)-1-(2,4-dichlorophenyl)-4-methyl-4,5-dihydro-1H-pyrazole-3-carboxylic acid, useful as intermediate in the preparation of cannabinoid CB1 neutral antagonists**

(71) Applicant: Esteve Química, S.A., 08024 Barcelona (ES)
(72) Inventor: Bartra Sanmartí, Martí, 08024, BARCELONA (ES); Berenguer Maimó, Ramon, 08024, BARCELONA (ES); Lluís Tous, Jordi, 08024, BARCELONA (ES)
(74) Representative: Ponti Sales, Adelaida

(57) **Abstract**

The present invention relates to a cinchonidine salt of (4S,5S)-5-(4-chlorophenyl)-1-(2,4-dichlorophenyl)-4-methyl-4,5-dihydro-1H-pyrazole-3-carboxylic acid. This cinchonidine salt is prepared by enantiomeric resolution of a compound of formula (I) comprising: 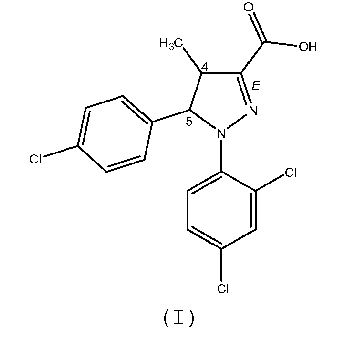 the reaction of compound of formula (I) with cinchonidine in a suitable solvent, thereby resulting in the cinchonidine salt of the enantiomer (4S,5S) of formula (II); and optionally isolating said salt. The cinchonidine salt according to formula (II) is a useful intermediate in the preparation of a cannabinoid CB₁ neutral antagonist.

## Description

### Field of the invention

The invention relates to organic chemistry, and in particular to reagents and synthetic routes for preparing pharmaceutical compounds.

### Background of the invention

Diseases characterised by impaired energy balance, such as obesity, are among the leading causes of illness and mortality in developed countries. It has been documented that the cannabinoid system, including receptors CB₁ and CB₂, is involved in the regulation of food intake and appetite (Cani et al. 2004). The discovery of this system has prompted the development of CB₁- and CB₂-selective receptor agonists, mixed CB₁/CB₂ receptor agonists, and selective antagonists.

Patent publication WO07/009689 (Laboratorios del Dr. Esteve S.A.) discloses the racemate compound cis-5-(4-chlorophenyl)-1-(2,4-dichlorophenyl)-N-(2,6-dimethylpiperidin-1-yl)-4-methyl-4,5-dihydro-1H-pyrazole-3-carboxamide (see compound nr 103 on page 229). This denomination embraces 4 isomers resulting from the chiral carbon atoms 4 and 5 of the pyrazoline ring, and from the carbon atoms 2 and 6 of the piperidine ring. This racemate possesses CB₁ inverse agonistic activity and as such is responsible for undesirable side effects including depressive disorders, anxiety, mood alterations with depressive symptoms, nausea, and dizziness.

It has been surprisingly found that the compound *(4S,5S)-*5-(4-chlorophenyl)-1-(2,4-dichlorophenyl)-N-(2,6-dimethylpiperidin-1-yl)-4-methyl-4,5-dihydro-1H-pyrazole-3-carboxamide, referred herein as compound of formula (V), is a promising CB₁ neutral antagonist, not inverse agonist, useful for example in the treatment of obesity but without exhibiting the previously mentioned undesirable side effects. These teachings are described in patent application PCT/EP2009/054175 (Laboratorios del Dr. Esteve), not yet published at the time of filing this application.

Optimized synthetic routes and valuable intermediates for upscaling the production of compound of formula (V) are desirable. With this in mind, and considering the stereoisomery of compound of formula (V), enantiomeric excesses and high yields are to be considered as well as the use of simplified processes and cost-effective reaction conditions that can result in savings in energy, time, installations, or personnel dedication.

WO07/009689 provides a method for the preparation of compound nr. 103, as a mixture of at least two isomers resulting from the chiral carbon atoms at 4 and 5 of the pyrazoline ring, with its undesirable effects. An intermediate for the preparation of compound nr 103 is the racemate cis-5-(4-chlorophenyl)-1-(2,4-dichlorophenyl)-4-methyl-4,5-dihydro-1H-pyrazole-3-carboxylic acid. This compound is prepared by reacting 4-(4-chlorophenyl)-3-methyl-2-oxo-3-butenoic acid with 2,4-dichlorophenyl hydrazine or a salt thereof in acetic acid, and heating to allow cyclization and formation of the pyrazoline ring, followed by removal of its trans isomer, for example, through separation of the methyl ester derivative and hydrolysis thereof (see page 173).

Among the multiple possibilities for devising an optimized synthetic route, the inventors have surprisingly found that optical resolution of the racemate cis-5-(4-chlorophenyl)-1-(2,4-dichlorophenyl)-4-methyl-4,5-dihydro-1H-pyrazole-3-carboxylic acid by diastereomeric salt formation with the chiral base cinchonidine results in a valuable intermediate for the preparation of compound of formula (V). This optical resolution and the obtained intermediate present one or more of the following advantages: they allow to obtain the desired (*4S,5S*) 5-(4-chlorophenyl)-1-(2,4-dichlorophenyl)-4-methyl-4,5-dihydro-1H-pyrazole-3-carboxylic acid from its racemate cis-5-(4-chlorophenyl)-1-(2,4-dichlorophenyl)-4-methyl-4,5-dihydro-1H-pyrazole-3-carboxylic acid with good enantiomeric excess and high yield, they allow a further one-pot procedure for the preparation of compound of formula (V), diminishing the subsequent number of processing steps and the amounts of reagents. Importantly, the optical resolution results in the synthesis of an intermediate in superior yields and enantiomeric excess. This enantiomeric resolution is also practical since it results in the precipitation of the desired diastereomeric salt, the one of the enantiomer (*4S,5S*)-, which can be further processed, while the non-desired diastereomeric salt, i.e. the enantiomer (*4R,5R*)-, remains in solution and can be easily separated.

Since the teachings regarding compound (V) and its pharmacological activities are not available in the state of the art, the envisioning and design of an optimal synthetic route as well as valuable intermediates for the production of compound (V) is a non-existing technical problem. Nevertheless, the following argumentation defending the merits of the invention is added for completeness.

The employed process, optical resolution, is generally described in Jacques, et al., Enantiomers, Racemates, and Resolutions (John Wiley and Sons 1981). Also, for example EP1743892 (Laboratorios del Dr. Esteve) uses chiral bases to resolve by diastereomeric salt formation pyrazoline compounds with one chiral carbon at position 5.

Unfortunately, when attempting to apply these general or more specific teachings to efficiently resolve enantiomeric mixtures, it is impossible to foresee what conditions or resolving agents will be successful. It is also not predictable that the enantiomeric resolution of compounds with one, two, or more chiral centers will succeed in the actual crystallization of desired diastereomeric salts, let alone with the desired steroisomery, nor expect to obtain the diastereomeric salt in high yield and diastereomeric ratio. It may not be anticipated either which chiral base will induce crystallization, even less, of the desired diastereomeric salt. It may not be anticipated either which solvent will induce crystallization, let alone, of the desired diastereomeric salt. It is not foreseeable either whether solvation occurs during salt formation, which phenomenon may influence the equilibrium between the diastereomeric salts with differing stability. It is further not foreseeable which diastereomeric salt will be predominant in the precipitate and in which diastereomeric purity.

Optical resolution via diastereomeric salt formation is usually based on the separation of diastereomers by fractional crystallization (Newmann P. Optical resolution procedures for chemical compounds, Vol. 1-3, 1978-1984). These methods often entail 5-10 recrystallization steps, but, for example, in the case of the resolution of racemic α-chlorobutyric acid with cinchonidine as resolving agent, in one study the precipitated diastereoisomer salt needed to be recrystallized 159 times (Jacques et al., 1981). It is therefore desirable that the optical resolution via diastereomeric salt formation requires a minimum number of crystallizations.

The present invention provides a process of preparing the compounds of the present invention by disclosing a remarkably effective resolving agent that selectively precipitates the substantially pure diastereomeric salt of the enantiomer (*4S,5S*).

### Summary of the invention

The present invention relates to a cinchonidine salt of (*4S,5S*)-5-(4-chlorophenyl)-1-(2,4-dichlorophenyl)-4-methyl-4,5-dihydro-1H-pyrazole-3-carboxylic acid, as represented by formula (II), and the solvates thereof.

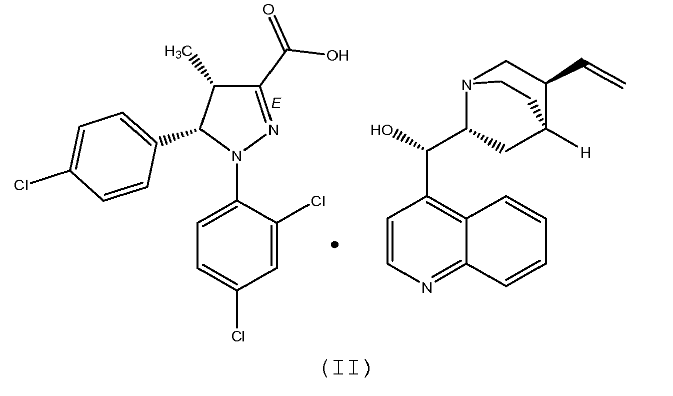

The present invention further relates to a method for the enantiomeric resolution of a compound of formula (I) comprising: 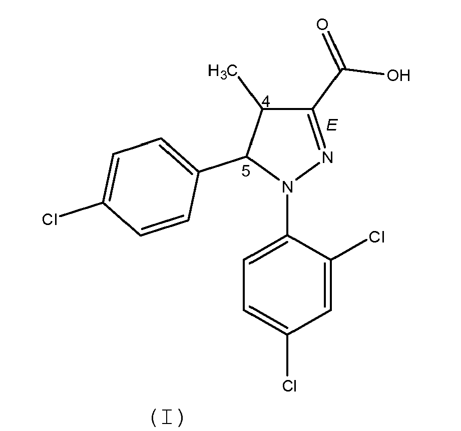 the reaction of compound of formula (I) with cinchonidine in a suitable solvent, thereby resulting in the cinchonidine salt of the enantiomer (4S,5S) of formula (II) as defined above; and optionally isolating said salt; wherein the carbon atoms 4 and 5 of the pyrazoline ring of compound of formula (I) present a relative cis configuration.

The cinchonidine salt according to formula (II) is a useful intermediate in the preparation of the compound of formula (III), (IV), (V), or a pharmaceutically acceptable salt thereof, as defined herein.

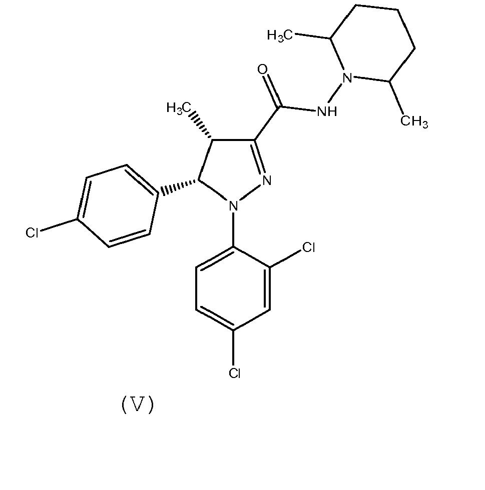

### Disclosure of the invention

The present invention relates to a cinchonidine salt of (*4S,5S*)-5-(4-chlorophenyl)-1-(2,4-dichlorophenyl)-4-methyl-4,5-dihydro-1H-pyrazole-3-carboxylic acid, as represented by formula (II), and the solvates thereof.

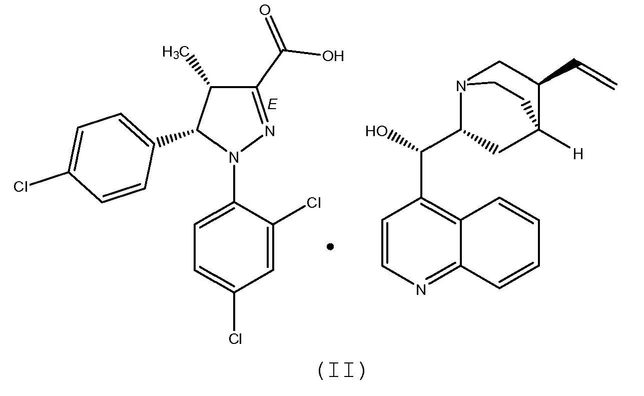

The term "solvate" refers to those forms of the compound of formula (II) that contain either stoichiometric or non-stoichiometric amounts of solvent. Since water is a solvent, solvates also include hydrates.

The present invention relates as well to the use of the cinchonidine salt, as represented by formula (II), as an intermediate in the preparation of the compound of formula (III), (IV), (V), or a pharmaceutically acceptable salt thereof, as defined herein.

The present invention relates to methods for the preparation of the compounds of formula (II), (III), (IV), and (V). The following scheme 1 illustrates the methods of the present invention.

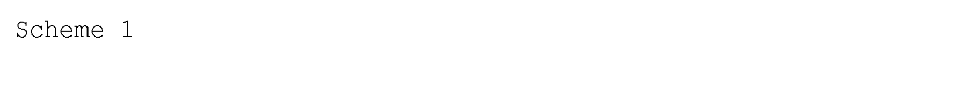

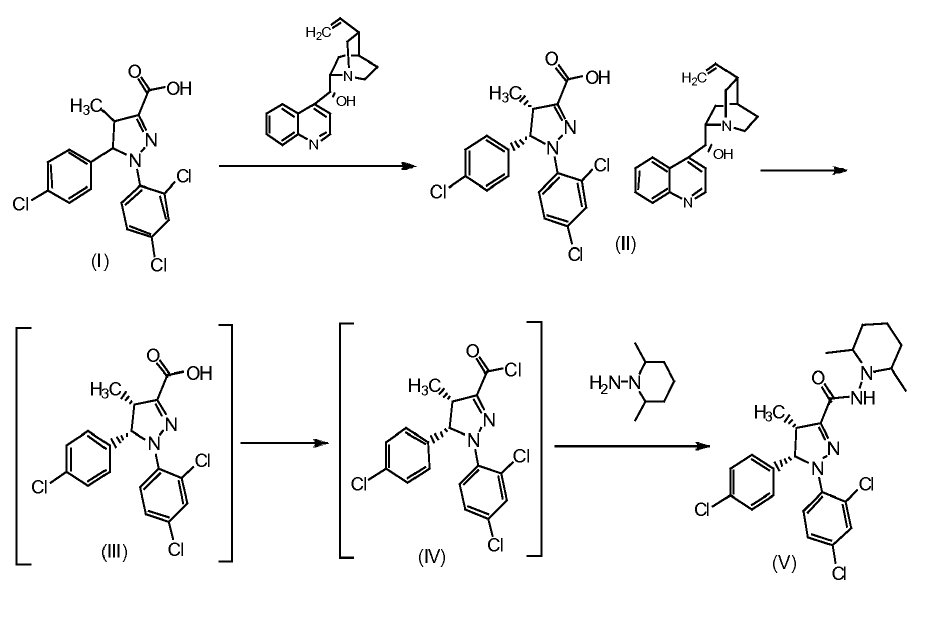

Thus, in one embodiment, the present invention relates to a method for the enantiomeric resolution of a compound of formula (I) comprising: 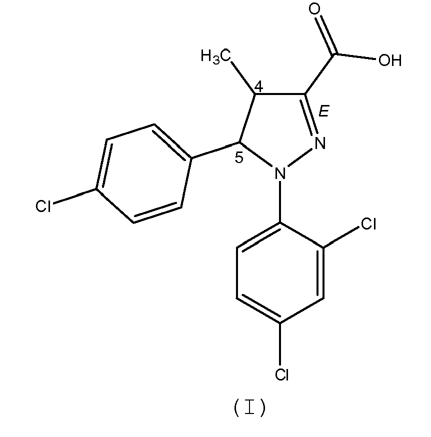 the reaction of compound of formula (I) with cinchonidine in a suitable solvent, thereby resulting in the cinchonidine salt of the enantiomer (4S,5S) of formula (II) as defined in above; and optionally isolating said salt; wherein the carbon atoms 4 and 5 of the pyrazoline ring of compound of formula (I) present a relative cis configuration.

The term "relative cis configuration" in the context of the carbon atoms 4 and 5 of the pyrazoline ring of compound of formula (I) refers to two possible alternative compounds as depicted below:

| | |
|---|---|
| 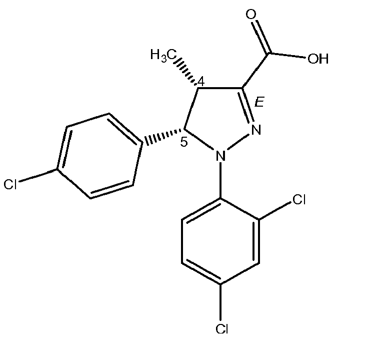 | 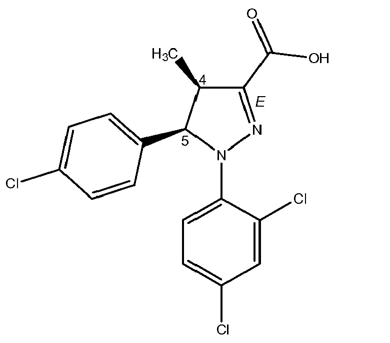 |
| (*4S,5S*)-5-(4-chlorophenyl)-1-(2,4-dichlorophenyl)-4-methyl-4,5-dihydro-1H-pyrazole-3-carboxylic acid | (*4R,5R*)-5-(4-chlorophenyl)-1-(2,4-dichlorophenyl)-4-methyl-4,5-dihydro-1H-pyrazole-3-carboxylic acid |

The starting material, i.e. compound of formula (I), is a mixture of the (*4S,5S*)- and (*4R,5R*)- isomers, in any proportion possible such as 50:50, 60:40, 70:30, 75:25, 80:20, 85:15, 90:10, 95:5. This mixture present in compound of formula (I) can be prepared as described in WO07/009689. It can be obtained from reacting 4-(4-chlorophenyl)-3-methyl-2-oxo-3-butenoic acid with 2,4-dichlorophenyl hydrazine or a salt thereof in acetic acid, and heating to allow cyclisation and formation of the pyrazoline ring.

The suitable solvent is preferably selected from methanol, methyl t*ert*-butyl ether (MTBE), and mixtures thereof.

In the preparation of the cinchonidine salt, the amount in mL of the solvent per gram of compound of formula (I) depends on the solvent used and can be determined by a person skilled in the art. For instance, in the case of methanol, the amount of solvent per gram of compound of formula (I) and cinchonidine may range from 0.1 mL to 50 ml, preferably from 0.5 to 25 ml, more preferably from 1 to 10 mL. In the case of MTBE, the amount of solvent per gram of compound of formula (I) may range from 1 to 100 mL, preferably from 5 to 75 mL, more preferably from 10 to 50 mL.

In the preparation of the cinchonidine salt using mixtures of methanol and MTBE, the ratio methanol/MTBE can also be readily determined by a person skilled in the art. For example, the mixtures of methanol and MTBE may range in the ratios 0.01/1 to 5/1, preferably from 0.05/1 to 2.5/1, more preferably from 0.08/1 to 2/1.

In carrying out the diastereomeric resolution of the present invention, a molar equivalent of cinchonidine to compound of formula (I) may be used in this reaction. However, it should be understood that other proportions may be used, such as from 0.5 to 2 molar equivalents of cinchonidine, preferably from 0.5 to 1.25 equivalents, more preferably from 0.5 to 1.1 molar equivalents of cinchonidine.

At the conclusion of the reaction, the diastereomeric salt is separated from the reaction mixture. In this context, the term "isolating said salt" refers to separating the cinchonidine salt of (*4S,5S*)-5-(4-chlorophenyl)-1-(2,4-dichlorophenyl)-4-methyl-4,5-dihydro-1H-pyrazole-3-carboxylic acid that crystallizes from the other components of the mixture. Isolation of the referred salt may be performed, for example, by filtration.

In one embodiment, the reaction of compound of formula (I) with cinchonidine in a suitable solvent, thereby resulting in the cinchonidine salt of the enantiomer (4S,5S) of formula (II); and optionally isolating said salt; is further followed by the crystallization of compound of formula (II) in a suitable solvent, and optionally isolating said salt.

Preferably the suitable solvent to perform a crystallization of the cinchonidine salt of the enantiomer (4S,5S) of formula (II) is selected from methanol, methyl tert-butyl ether (MTBE), and mixtures thereof.

In one embodiment, the cinchonidine salt of the enantiomer *(4S,5S)* of formula (II) is first isolated by filtration and can be further crystallized to improve its diastereomeric ratio. This crystallization step could be repeated more than once to continue improving its diastereomeric ratio.

In one embodiment, any one of the methods of the present invention further comprises removing the cinchonidine by reacting compound of formula (II) with a suitable acid, thereby obtaining compound of formula (III).

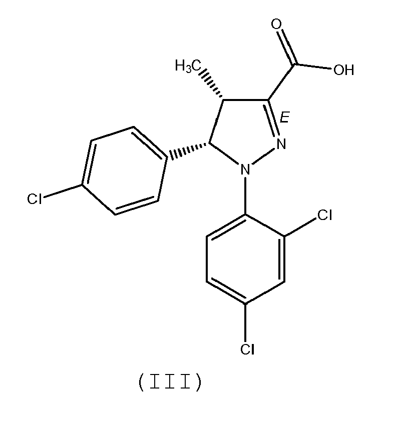

The term "suitable acid" refers to those acids able to remove the cinchonidine salt without changing the chemical composition of (*4S,5S*)-5-(4-chlorophenyl)-1-(2,4-dichlorophenyl)-4-methyl-4,5-dihydro-1H-pyrazole-3-carboxylic acid. The suitable acid may be selected from, without being limited to, hydrohalic acids, e.g. hydrochloric or hydrobromic acid, sulfuric, nitric, phosphoric and the like acids; or organic acids such as, for example, acetic and the like acids.

In one embodiment, after removal of the cinchonidine, the methods comprise converting compound of formula (III) into a compound of formula (IV) with an activating agent, thereby obtaining compound of formula (IV) 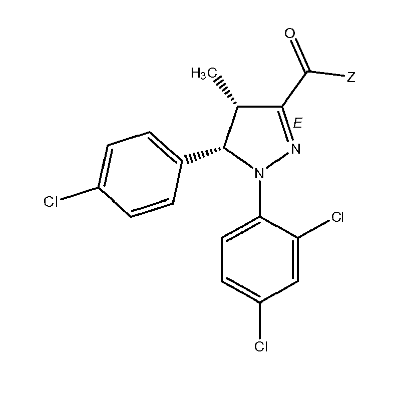 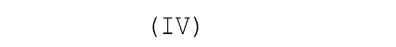 wherein
**Z** represents halo, -O-CO-R¹, or -O-CO-OR¹; and
**R¹** is C₁-₄alkyl, aryl, or benzyl.

In another embodiment, the methods further comprise coupling said compound of formula (IV) with 2,6-dimethylaminopiperidine in a suitable solvent, thereby obtaining a compound of formula (V).

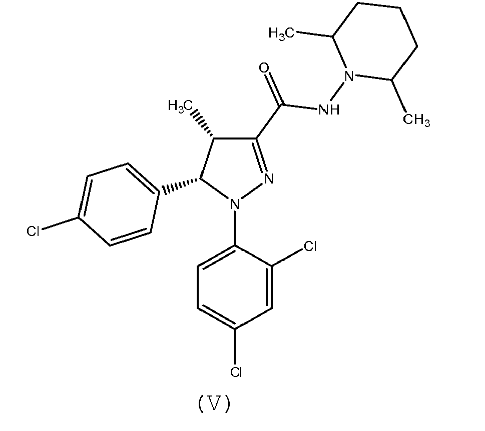

Alternatively, after removal of the cinchonidine, the methods comprise coupling compound of formula (III) with 2,6-dimethylaminopiperidine in a suitable solvent, in the presence of a coupling agent, and optionally in the presence of an activating agent of the coupling agent according to the methods known by the skilled in the art, thereby obtaining a compound of formula (V).

The term "halo" is generic to fluoro, chloro, bromo, and iodo.

The term "C₁-₄alkyl" as a group or part of a group defines straight or branched chain saturated hydrocarbon radicals having from 1 to 4 carbon atoms such as methyl, ethyl, propyl, 1-methylethyl, and butyl.

The term "aryl" as a group or part of a group is phenyl or naphthyl.

The coupling reactions of each of steps a1) and a2) are conducted according to the methods known by the skilled in the art in a suitable solvent. Such suitable solvent is usually of inert nature, such as toluene; halogenated hydrocarbons, e.g. dichloromethane, 1,2-dichloroethane, and chloroform; dipolar aprotic solvents such as acetonitrile, dimethylformamide, and dimethylacemide; ethers such as tetrahydrofuran and diethylether; and the like.

According to the process for the preparation of compound of formula (V), compound of formula (III) may be converted into an activated form, i.e. compound of formula (IV), with the use of an activating agent according to the methods known by the skilled in the art. Such activating agent may be selected from, without being limited to, a halogenating agent, C₁₋₄alkyl acid halide, aryl acid halide, benzyl acid halide, C₁₋₄alkyl haloformate, aryl haloformate, and benzyl haloformate.

Examples of halogenating agents that may be used include, but are not limited to, inorganic acid halides, thionyl chloride, cyanuric chloride, Vilsmeier reagent, Gold's reagent, chlorinated heterocycles, and combinations of halogenating agents such as halogens, CCl₄, C₂Cl₆, or other alkyl halides with reducing agents such as triaryl or trialkyl phosphines or phosphites or a hydrogen halide in the presence of a dehydrating agent. Examples of C₁₋₄alkyl acid halides include acetyl chloride, propionyl chloride, butanoyl chloride, and the like. Examples of aryl acid halides include phthaloyl chloride, isophthaloyl chloride, and terphthaloyl chloride. Examples of benzyl acid halides include benzoyl chloride, benzoyl fluoride, benzoyl bromide. Examples of C₁₋₄alkyl haloformate include methyl chloroformate, ethyl chloroformate, isopropyl chloro-formate, and the like. Examples of aryl haloformate include phenyl chloroformate, phenyl fluoroformate, and the like. Examples of benzyl haloformates include benzyl chloroformate, benzyl fluoroformate, and the like. Examples of reagents useful for the conversion of carboxylic acids to acid halides or acid anhydrides are listed in the book Comprehensive Organic Transformations 2nd Edition, Richard C. Larock, pp 1929-1932, which is incorporated herein by reference.

*Cis*-2,6-dimethyl-1-piperidinamine (CAS registry nr 61147-58-8) and *cis*/*trans*-2,6-dimethylpiperidine (CAS registry nr 39135-39-2) are commercially available for example from Fluka, 3B Scientific Corporation, Pfaltz & Bauer Chemicals, Sigma-Aldrich, Amfinecom, among other. *Trans-*2,6-dimethylpiperidine (CAS registry nr 876307-54-9) is disclosed in Jucker et al. (Helvetica Chimica Acta, 1962, 45 (7), 2316-25).

In one embodiment, compound of formula (V) is further reacted with a pharmaceutically acceptable acid thereby obtaining the salt of compound of formula (V).

The pharmaceutically acceptable acid addition salts as mentioned hereinabove are meant to comprise the therapeutically active non-toxic acid addition salt forms which the compounds of formula (V) are able to form. The pharmaceutically acceptable acid addition salts can conveniently be obtained by treating the base form with such appropriate acid. Appropriate acids comprise, for example, the anions acetate, benzenesulfonate , benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, and triethiodide. Preferably, the salt is formed with hydrochloric acid.

Once compound of formula (V) is prepared, it is conveniently separated from the reaction mixture. In one embodiment, compound of formula (V) is isolated by crystallization, optionally adding an anti-solvent, and subsequent filtration. Suitable anti-solvents include alcohols, such as ethanol, methanol, isopropanol, and the like, amongst others. This separation procedure is particularly advantageous when compared to those ones using chromatography columns.

The methods of the present invention may be performed in a one-pot procedural format, particularly, the conversion of compound of formula (II) into (V), since compounds of formula (III) and (IV) may remain in solution in the same reaction vessel, without needing to be isolated or purified.

In one embodiment, this one-pot procedure is performed by using the same solvent medium throughout the reactions. Preferably, the solvent used in the one-pot procedure, i.e. the conversion of compound of formula (II) to (III) to (IV) to (V), is toluene.

The following examples are intended to illustrate the present invention and not to limit it thereto.

### Examples

**Example 1: Preparation of (*4S,5S*)-5-(4-chlorophenyl)-1-(2,4-dichlorophenyl)-4-methyl-4,5-dihydro-1H-pyrazole-3-carboxylic acid-Cinchonidine salt in methanol** A three-neck 250 ml flask was charged with 45.0 g of 5-(4-chlorophenyl)-1-(2,4-dichlorophenyl)-4-methyl-4,5-dihydro-1H-pyrazole-3-carboxylic acid (1.00 eq) and 81 ml of methanol. 36.3 g of *(-)-Cinchonidine* (1.05 eq) were added. The reaction mixture was heated and maintained for 15 minutes at reflux temperature. The solution was cooled to 20-30 °C and it was filtered to obtain a clear solution. The solution was cooled to 15-20 °C and seeded with (*4S,5S*)-5-(4-chlorophenyl)-1-(2,4-dichlorophenyl)-4-methyl-4,5-dihydro-1H-pyrazole-3-carboxylic acid-Cinchonidine salt. To the seeded solution, 40.5 ml of fresh methanol were added for about 15 minutes at 15-20 °C. During the addition of the methanol (*4S,5S*)-5-(4-chlorophenyl)-1-(2,4-dichlorophenyl)-4-methyl-4,5-dihydro-1-pyrazole-3-carboxylic acid-Cinchonidine salt started to precipitate. It was maintained for about 30 minutes at 15-20 °C. The dispersion was cooled to -10 °C and maintained for over 6 hours. The solid product was isolated by filtration and washed twice with 24 ml of cold methanol. The solid was dried under vacuum at 60 °C to obtain 29.0 g of (*4S,5S*)-5-(4-chlorophenyl)-1-(2,4-dichlorophenyl)-4-methyl-4,5-dihydro-1H-pyrazole-3-carboxylic acid-Cinchonidine salt as a white product (36 %).
Diastereomeric excess (d.e.): 97.8 % by chiral High Performance Liquid Chromatography (HPLC).

### Example 2: Crystallization of (4S,5S)-5-(4-chlorophenyl)-1-(2,4-dichlorophenyl)-4-methyl-4,5-dihydro-1H-pyrazole-3-carboxylic acid-Cinchonidine salt in methanol

320 g of a mixture of (*4S,5S*)-5-(4-chlorophenyl)-1-(2,4-dichlorophenyl)-4-methyl-4,5-dihydro-1H-pyrazole-3-carboxylic acid-Cinchonidine salt (d.e. 88.1 % by quiral HPLC) was dissolved in 1600 ml of methanol at 25-35°C. The resulting solution was filtered through Celite ® and the filtrate was seeded. The solid crystallized and the slurry was maintained for about 30 minutes at 25-35°C. Then it was cooled to 0-5 °C and maintained for over 1 hour. The product was isolated by filtration and washed twice with 96 ml of cold methanol. The product was dried at 60 °C under vacuum obtaining 264 g of (*4S,5S*)-5-(4-chlorophenyl)-1-(2,4-dichlorophenyl)-4-methyl-4,5-dihydro-1-pyrazole-3-carboxylic acid-Cinchonidine salt as a white solid (82 %).
d.e.: 99.9 % by chiral HPLC.

**Example 3: Preparation of (4S,5S)-5-(4-chlorophenyl)-1-(2,4-dichlorophenyl)-4-methyl-4,5-dihydro-1H-pyrazole-3-carboxylic acid-Cinchonidine salt in methyl tert-butyl ether (MTBE)** A three-neck 250 ml flask was charged with 5.0 g of 5-(4-chlorophenyl)-1-(2,4-dichlorophenyl)-4-methyl-4,5-dihydro-1H-pyrazole-3-carboxylic acid (1.0 eq) and 1.9 g of *(-)-Cinchonidine* (0.5 eq). 138 ml of MTBE were added. The reaction mixture was heated and maintained for over 20 minutes at reflux temperature. The solution was seeded with (*4S,5S*)-5-(4-chlorophenyl)-1-(2,4-dichlorophenyl)-4-methyl-4,5-dihydro-1H-pyrazole-3-carboxylic acid-Cinchonidine salt and slowly cooled to room temperature. At about 29 °C white solid crystallizes and it was maintained for about 30 minutes at this temperature. The dispersion was cooled to 0-5 °C and maintained for about 1 hour. The solid product was isolated by filtration and washed twice with 2.6 ml of cold MTBE. The solid was dried under vacuum at 40 °C to obtain 3.2 g of (*4S,5S*)-5-(4-chlorophenyl)-1-(2,4-dichlorophenyl)-4-methyl-4,5-dihydro-1H-pyrazole-3-carboxylic acid-Cinchonidine salt as a white solid (36 %). d.e.: 84.4 % by chiral HPLC.

### Example 4: Crystallization of (4S,5S)-5-(4-chlorophenyl)-1-(2,4-dichlorophenyl)-4-methyl-4,5-dihydro-1H-pyrazole-3-carboxylic acid-Cinchonidine salt in methyl tert-butyl ether (MTBE)

2.5 g of (*4S,5S*)-5-(4-chlorophenyl)-1-(2,4-dichlorophenyl)-4-methyl-4,5-dihydro-1H-pyrazole-3-carboxylic acid-Cinchonidine salt (obtained by quiral HPLC, d.e. 93.8 %) was dispersed in 82 ml of MTBE at reflux temperature. It was maintained for over 15 minutes at reflux temperature. The dispersion was slowly cooled to room temperature. The product was isolated by filtration and washed twice with 0.8 ml MTBE. The product was dried at 40 °C under vacuum obtaining 2.4 g of (*4S,5S*)-5-(4-chlorophenyl)-1-(2,4-dichlorophenyl)-4-methyl-4,5-dihydro-1H-pyrazole-3-carboxylic acid-Cinchonidine salt (94 %).
d.e.: 99.9 % by chiral HPLC.

### Example 5: Preparation of (4S,5S)-5-(4-chlorophenyl)-1-(2,4-dichlorophenyl)-N-(2,6-dimethylpiperidin-1-yl)-4-methyl-4,5-dihydro-1H-pyrazole-3-carboxamide

A/ Preparation of (*4S,5S*)-5-(4-chlorophenyl)-1-(2,4-dichlorophenyl)-4-methyl-4,5-dihydro-1H-pyrazole-3-carboxylic acid from its Cinchonidine salt A 3 1 reactor was charged with 264 g of (*4S,5S*)-5-(4-chlorophenyl)-1-(2,4-dichlorophenyl)-4-methyl-4,5-dihydro-1H-pyrazole-3-carboxylic acid-Cinchonidine salt (1.0 eq) and 1320 ml of toluene were added at room temperature. To the solution at room temperature 132 ml of water were added. The solution was acidified to pH 1 with 75 ml of concentrated hydrochloric acid. The aqueous layer was decanted, and the organic layer was washed with 132 ml of water. The organic layer was concentrated to about 3/4 parts of the initial volume under vacuum at temperature below 80 °C (149 g of (*4S,5S*)-(4-chlorophenyl)-1-(2,4-dichlorophenyl)-4-methyl-4,5-dihydro-1H-pyrazole-3-carboxylic acid, theoretical in toluene).

### B/ Preparation of (4S,5S)-5-(4-chlorophenyl)-1-(2,4-dichlorophenyl)-4-methyl-4,5-dihydro-1H-pyrazole-3-carbonyl chloride from (4S,5S)-5-(4-chlorophenyl)-1-(2,4-dichlorophenyl)-4-methyl-4,5-dihydro-1H-pyrazole-3-carboxylic acid

A 3 1 reactor was charged with a solution of (4*S*,5*S*)-5-(4-chlorophenyl)-1-(2,4-dichlorophenyl)-4-methyl-4,5-dihydro-1H-pyrazole-3-carboxylic acid in toluene (1.0 eq, 149 g theoretical) and 34 ml of thionyl chloride (SOCl₂, 1.2 eq). The reaction mixture was heated and maintained for over 2 h 30 minutes at about 80 °C. 320 ml of toluene were distilled under vacuum at temperature below 80 °C. 320 ml of fresh toluene were added. Finally 610 ml of toluene were distilled under vacuum at temperature below 80 °C. (156 g of theoretical product in toluene).

C/ Preparation of (*4S,5S*)-5-(4-chlorophenyl)-1-(2,4-dichlorophenyl)-N-(2,6-dimethylpiperidin-1-yl)-4-methyl-4,5-dihydro-1H-pyrazole-3-carboxamide from (*4S,5S*)-5-(4-chlorophenyl)-1-(2,4-dichlorophenyl)-4-methyl-4,5-dihydro-1H-pyrazole-3-carbonyl chloride A 3 1 reactor was charged with 52.4 g of 1-amino-cis-2,6-dimethylpiperidine (1.05 eq). 780 ml of toluene and 81.4 ml of *N,N-*Diisopropylethylamine (DIPEA, 1.2 eq) were added. The reaction mixture was cooled to 0-5 °C. A solution of (*4S,5S*)-5-(4-chlorophenyl)-1-(2,4-dichlorophenyl)-4-methyl-4,5-dihydro-1H-pyrazole-3-carbonyl chloridein toluene (1.0 eq, 156 g theoretical) was added in about 40 minutes at 0-5 °C. The solution was left to warm to room temperature and maintained for over 2 hours.

To the solution at room temperature 312 ml of water and 151 ml of acetic acid were added. The aqueous layer was decanted (pH 4), and the organic layer was washed again with 312 ml of water and 151 ml of acetic acid. The aqueous layer was decanted (pH 4), and the organic layer was cooled to 0-5 °C. The organic layer was basified to pH 8-9 with 312 ml of a saturated solution of aqueous sodium bicarbonate. The aqueous layer was decanted. The organic layer was warmed to room temperature and it was washed with 624 ml of water. The aqueous layer was decanted (pH 7). The organic layer was concentrated to about 1/5 parts of the initial volume under vacuum at temperature about 50-60 °C. (192 g of theoretical product in toluene).

### D/ Crystallization of (4S,5S)-5-(4-chlorophenyl)-1-(2,4-dichlorophenyl)-N-(2,6-dimethylpiperidin-1-yl)-4-methyl-4,5-dihydro-1H-pyrazole-3-carboxamide

To a solution of (*4S,5S*)-5-(4-chlorophenyl)-1-(2,4-dichlorophenyl)-N-(2,6-dimethylpiperidin-1-yl)-4-methyl-4,5-dihydro-1H-pyrazole-3-carboxamide in toluene(384 g theoretical) 1820 ml of ethanol were added over 50 °C. The warm solution was filtered to obtain a clear solution. The solution was cooled to 35-40 °C and seeded. The solid crystallized at about 35 °C. The slurry was maintained for 30 minutes at this temperature. Then it was cooled at 0-5 °C and it was maintained for about 2 hours at this temperature. The product was isolated by filtration and washed with 110 ml of cold ethanol. The product was dried at 60 °C under vacuum obtaining 271 g of (*4S,5S*)-5-(4-chlorophenyl)-1-(2,4-dichlorophenyl)-N-(2,6-dimethylpiperidin-1-yl)-4-methyl-4,5-dihydro-1H-pyrazole-3-carboxamide as a white solid (71 % global yield from (4S,5S)-5-(4-chlorophenyl)-1-(2,4-dichlorophenyl)-4-methyl-4,5-dihydro-1H-pyrazole-3-carboxylic acid-Cinchonidine salt).
Enantiomeric excess (e.e.): 100 % by chiral HPLC.
Water content: 0.04 % w/w.
IR (KBr, cm⁻¹): 3271, 2923, 1658, 1526, 1478, 1305, 1269, 1118, 1093, 809.
¹H-NMR (CD₃OD, 400 MHz) δ (ppm): 7.45 (1H, d, 8.8 Hz), 7.35 (1H, d, 2.0 Hz), 7.25 (2H, m), 7.16 (3H, m), 5.86 (1H, d, 11.2 Hz), 3.85 (1H, m), 2.60 (2H, wide signal), 1.71-1.64 (3H, m), 1.51-1.34 (3H, m), 1.07 (3H, d, 6.4 Hz), 1.06 (3H, d, 6.0 Hz), 0.88 (3H, d, 7.2 Hz).
PXRD: 5.25, 7.86, 12.84, 14.83, 15.80, 18.40, 19.23, 20.34 ± 0.2 degrees 2θ.

## Claims

1. A cinchonidine salt of (*4S,5S*)-5-(4-chlorophenyl)-1-(2,4-dichlorophenyl)-4-methyl-4,5-dihydro-1H-pyrazole-3-carboxylic acid, as represented by formula (II), and the solvates thereof.

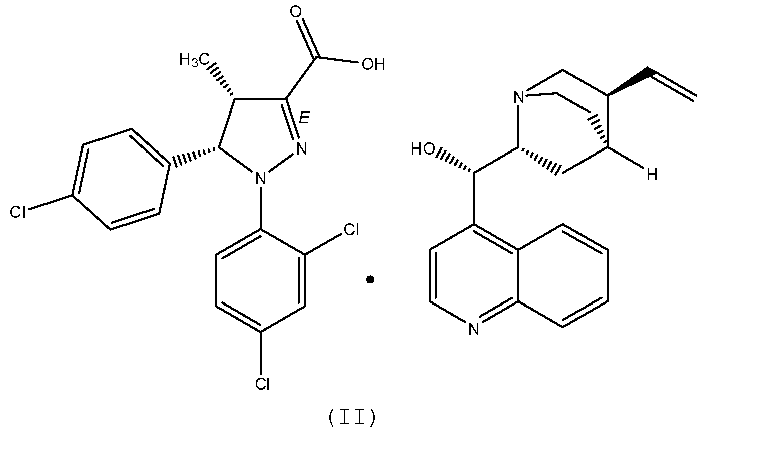

2. A method for the enantiomeric resolution of a compound of formula (I) comprising: 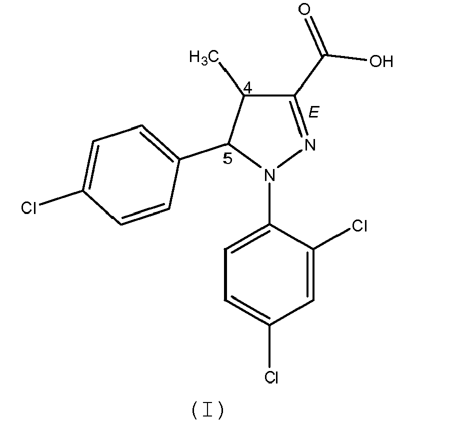 the reaction of compound of formula (I) with cinchonidine in a suitable solvent, thereby resulting in the cinchonidine salt of the enantiomer (4S,5S) of formula (II) as defined in claim 1; and optionally isolating said salt; wherein the carbon atoms 4 and 5 of the pyrazoline ring of compound of formula (I) present a relative cis configuration.

3. The method according to claim 2, wherein the suitable solvent is selected from methanol, methyl tert-butyl ether (MTBE), and mixtures thereof.

4. The method according to any one of claims 2-3, further comprising the crystallization of compound of formula (II) in a suitable solvent, and optionally isolating said salt.

5. The method according to claim 4, wherein the suitable solvent is selected from methanol, methyl tert-butyl ether (MTBE), and mixtures thereof.

6. The method according to any one of claims 2-5, further removing the cinchonidine by reacting compound of formula (II) with a suitable acid, thereby obtaining compound of formula (III).

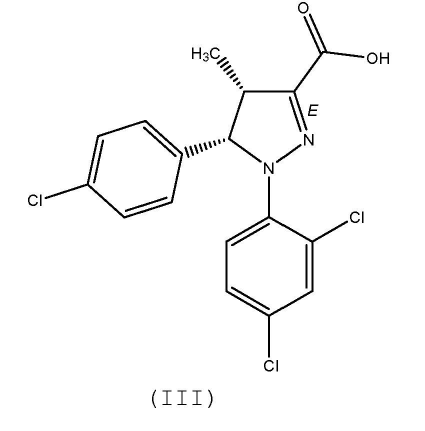

7. The method according to claim 6, further comprising converting compound of formula (III) into a compound of formula (IV) with an activating agent, thereby obtaining compound of formula (IV) 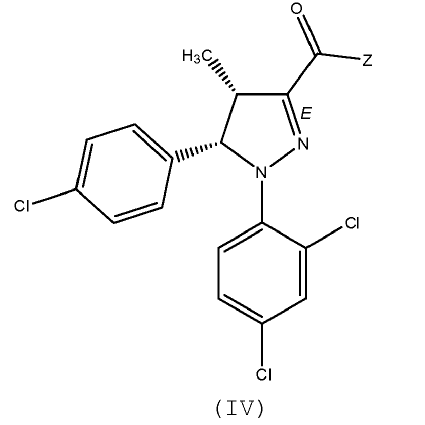 wherein
**Z** represents halo, -O-CO-R¹, or -O-CO-OR¹; and
**R¹** is C₁₋₄alkyl, aryl, or benzyl.

8. The method according to claim 7, further comprising coupling compound of formula (IV) with 2,6-dimethylaminopiperidine in a suitable solvent, thereby obtaining a compound of formula (V).

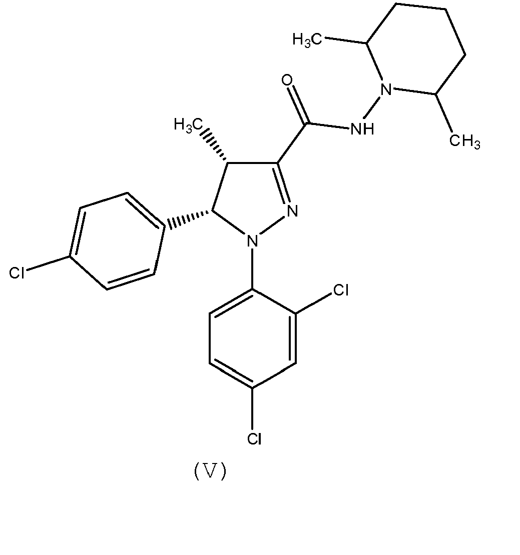

9. The method according to claim 8, further reacting compound of formula (V) with a pharmaceutically acceptable acid thereby obtaining the salt of compound of formula (V).

10. The method according to claim 9, wherein the pharmaceutically acceptable acid is hydrochloric acid.

11. The method according to any one of claims 8-10, wherein compound of formula (V) or the pharmaceutically acceptable thereof is isolated by crystallization, optionally adding an anti-solvent, and subsequent filtration.

12. The method according to any one of claims 6-11, wherein the methods are performed in a one-pot procedure.

13. The method according to any one of claims 6-12, wherein the solvent used is toluene.

14. Use of the cinchonidine salt according to claim 1 as an intermediate in the preparation of the compound of formula (III), (IV), (V), or a pharmaceutically acceptable salt thereof, as defined in claims 6, 7, or 8.
